(19) Europäisches Patentamt
European Patent Office
Office européen des brevets

(11) Veröffentlichungsnummer: **0 168 515**
**A2**

(12) **EUROPÄISCHE PATENTANMELDUNG**

(21) Anmeldenummer: **84108513.7**

(22) Anmeldetag: **19.07.84**

(51) Int. Cl.[4]: **C 07 C 50/36,** C 07 H 15/252, C 07 F 7/18

(43) Veröffentlichungstag der Anmeldung: **22.01.86 Patentblatt 86/4**

(84) Benannte Vertragsstaaten: **AT BE CH DE GB IT LI LU NL SE**

(71) Anmelder: **LABORATOIRES HOECHST S.A., Tour Roussel Nobel 3, Avenue du Général de Gaulle, F-92800 Puteaux (FR)**

(72) Erfinder: **Renoux, Brigitte, La Pierrière chez M. Broca, F-88820 St-Julien l'Ars (FR)**
Erfinder: **Gesson, Jean-Pierre, La Germanière mantamisé, F-86360 Chasseneuil du Poitou (FR)**
Erfinder: **Jean-Claude, Jacquesy, rue Planty 46, F-86180 Bruxerolles (FR)**

(74) Vertreter: **Reuter, Johann-Heinrich, Dr. et al, HOECHST AKTIENGESELLSCHAFT Zentrale Patentabteilung Postfach 80 03 20, D-6230 Frankfurt/Main 80 (DE)**

(54) **Neues Verfahren zur Herstellung von Steffimycinonanalogen, so erhaltene neue Steffimycinonanaloge, neue zur Synthese dieser Verbindungen verwendbare Zwischenprodukte und Verwendung dieser Verbindungen als Bakterizide und zur Synthese von Antitumormitteln.**

(57) Vorliegende Erfindung betrifft neue Steffimycinonanaloge und Verfahren zu ihrer Herstellung.

Diese Analogen entsprechen der nachfolgenden Formel II:

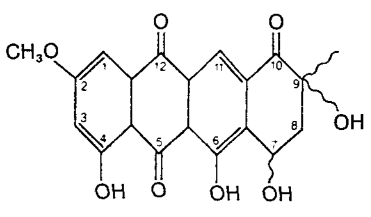

II

Verwendung als Bakterizide und zur Herstellung von Antitumormitteln durch Kupplung mit Zuckern.

EP 0 168 515 A2

LABORATOIRES HOECHST S.A. HOE 83/S 030 Dr. HA

# Neues Verfahren zur Herstellung von Steffimycinonanalogen, so erhaltene neue Steffimycinonanaloge, neue zur Synthese dieser Verbindungen verwendbare Zwischenprodukte und Verwendung dieser Verbindungen als Bakterizide und zur Synthese von Antitumormitteln

Vorliegende Erfindung betrifft ein neues Verfahren zur Synthese von Steffimycinonanalogen, so erhaltene neue Steffimycinonanaloge, neue zur Synthese dieser Verbindungen verwendbare Zwischenprodukte und die Verwendung dieser Verbindungen als Bakterizide und zur Synthese von Antitumormitteln.

Es gibt zahlreiche Antibiotika aus der Klasse der Anthracycline, welche der nachfolgenden allgemeinen Formel I entsprechen:

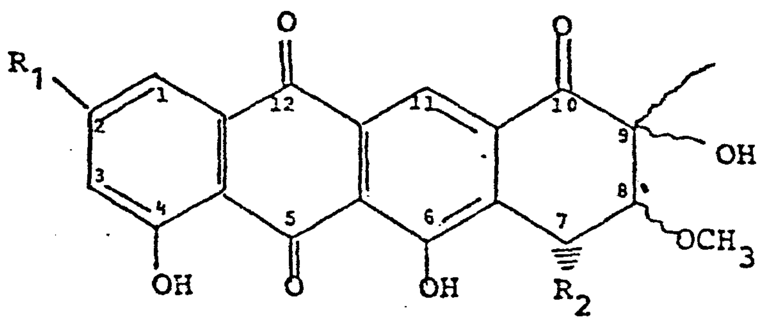

I

so zum Beispiel:

a) mit $R_1$ = $OCH_3$ und $R_2$ =

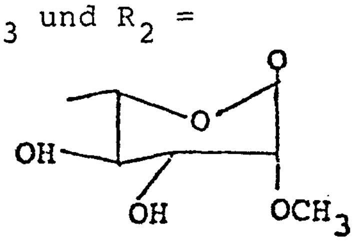

entspricht die Verbindung dem Steffimycin A (vgl. z.B. US Patente 3 309 273 und 3 976 667),

b) mit $R_1$ = $OCH_3$ und $R_2$ =

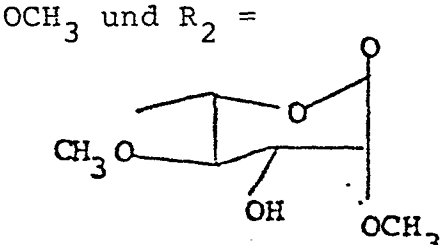

entspricht die Verbindung dem Steffimycin B (vgl. z.B. JACS 84, 1962, S. 3182),

c) mit $R_1$ = H und $R_2$ =

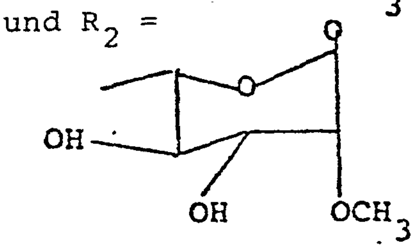

entspricht die erhaltene Verbindung dem Aranciamycin (vgl. z.B. JACS 84, 1962, S. 3182),

d) mit $R_1$ = H und $R_2$ = OH entspricht die erhaltene Verbindung dem Aranciamycinon (A. OGILVIE und Mitarbeiter in "Antibiotics", Herausgeber BAHN, Band V/I, S. 250) und

e) mit $R_1$ = H und $R_2$ = H entspricht die erhaltene Verbindung dem Antibiotikum SM 1731 B (Belgisches Patent 863 610).

Diese Verbindungen werden entweder direkt aus dem Fermentationsmedium oder durch saure Hydrolyse eines Fermentationsprodukts (beispielsweise im Fall des durch saure Hydrolyse von Aranciamycin erhaltenen Aglykons d)) erhalten.

Alle diese Verbindungen sind hochaktiv gegen grampositive Bakterien und besitzen zum Teil [insbesondere die Verbindungen a) und b)] cytotoxische Eigenschaften. Ferner besteht eine starke strukturelle Aehnlichkeit zwischen diesen verschiedenen Aglykonen und den Aglykonen gewisser, klinisch verwendeter Antitumormittel wie beispielsweise Daunomycin oder Adriamycin.

Es war die Aufgabe dieser Erfindung, einerseits ein neues Aglykonanalog des Steffimycinons zu schaffen und andererseits eine einfache und wirtschaftliche Synthese dieser Analogen vorzuschlagen, um somit für die Synthese von Antitumormitteln durch Kupplung mit Zuckern interessante Aglykone zur Verfügung zu haben.

Gegenstand vorliegender Erfindung sind Steffimycinonanaloge und insbesondere die 8-desmethoxylierten Derivate der nachfolgenden allgemeinen Formel II:

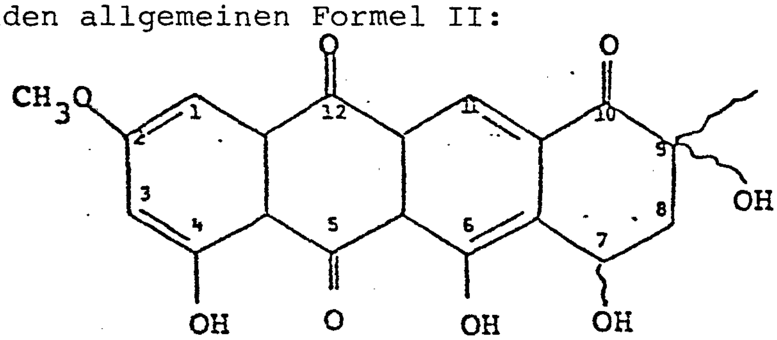

II

Erfindungsgemäss besteht dieses Analog aus (±)-8-Desmethoxy-steffimycinon.

Nach einer anderen Ausführungsform der Erfindung besteht dieses Analog aus (±)-7-Epi-8-desmethoxy-steffimycinon.

Ein weiterer Gegenstand dieser Erfindung ist ein Verfahren zur Herstellung von Steffimycinonanalogen der allgemeinen Formel II, welches durch die Kombination der folgenden Stufen gekennzeichnet ist:

1. Herstellung des silylierten Ketenacetals der nachfolgenden allgemeinen Formel III:

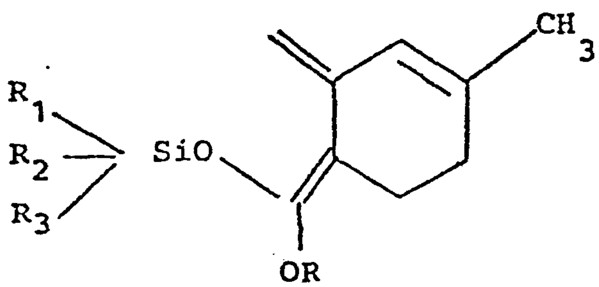

III

worin

R eine gegebenenfalls substituierte Alkyl- oder Arylgruppe sowie

$R_1$, $R_2$ und $R_3$, die gleich oder verschieden sein können, eine Alkylgruppe darstellen,

durch Umsetzung eines Lithiumamids und eines Silylierungsmittels mit einem Alkyl- oder Arylester der 2,4-Dimethyl-1,3-cyclohexadiencarbonsäure der nachfolgenden allgemeinen Formel IV:

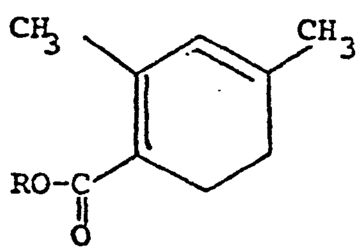

IV

wobei R die oben angegebene Bedeutung hat,

2. Herstellung des 5,7-Dihydroxy-9-methoxy-2-methyl-3,4-dihydro-6,11-naphthacendions der nachfolgenden Formel V:

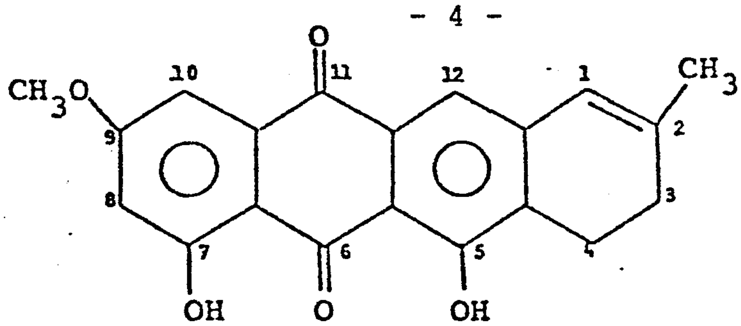

V

durch Umsetzung der Verbindung III mit einem 5-Hydroxy-7-methoxy-1,4-naphthochinon der nachfolgenden Formel VI:

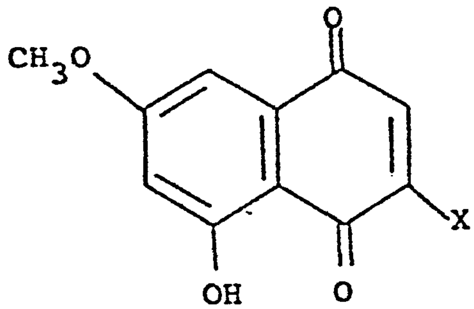

VI

worin

X für ein Halogenatom oder Wasserstoffatom steht,

3. Herstellung des (±)-5,7-Dihydroxy-1,2-epoxy-9-methoxy-2-methyl-1,2,3,4-tetrahydro-6,11-naphthacendions der nachfolgenden Formel VII:

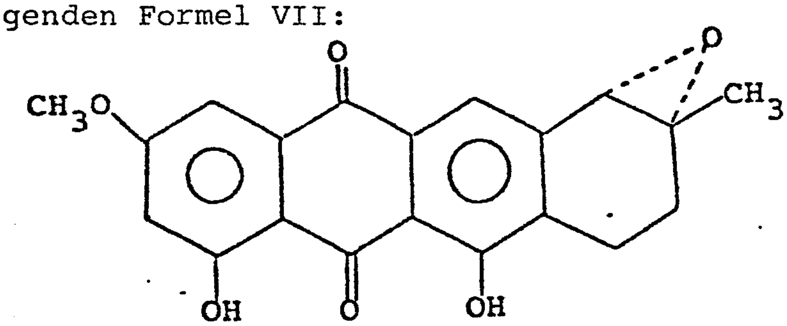

VII

durch Einwirkung einer Persäure auf das Olefin der Formel V,

4. Herstellung eines Gemischs der racemischen Diole [1α,2α und 1β,2α] der nachfolgenden Formel VIII:

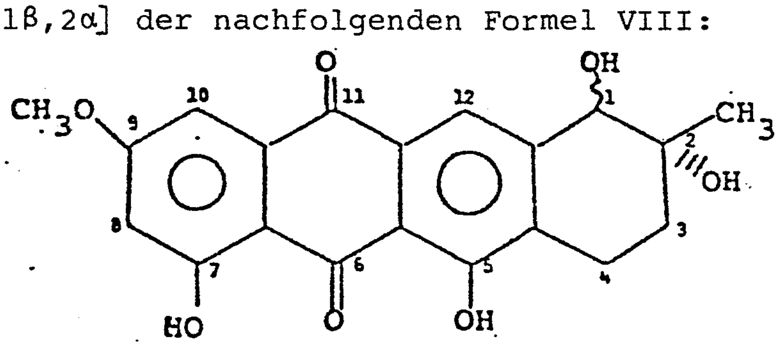

VIII

durch Behandlung des in der vorhergehenden Stufe erhaltenen Epoxyds mit einer Säure,

5. Herstellung des (±)-9-Methoxy-2-methyl-2,5,7-trihydroxy-3,4-dihydro-2H-1,6,11-naphthacentrions der nachfolgenden

Formel IX:

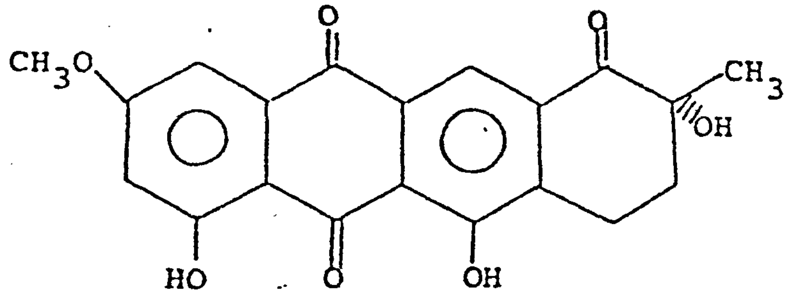

IX

durch Oxydation des in der vorhergehenden Stufe erhaltenen Gemischs aus

(±)-9-Methoxy-2-methyl-1α,2α,5,7-tetrahydroxy-1,2,3,4-tetrahydro-6,11-naphthacendion und

(±)-9-Methoxy-2-methyl-1β,2α,5,7-tetrahydroxy-1,2,3,4-tetrahydro-6,11-naphthacendion, und

6. Herstellung von 8-Desmethoxysteffimycinonanalogen der allgemeinen Formel II durch Behandlung des Produkts IX in einem polaren Lösungsmittel mit einer starken nicht-nukleophilen Base und nachfolgende saure Hydrolyse.

Nach einer Ausführungsform des Gegenstands der Erfindung lässt sich das (±)-9-Methoxy-2-methyl-1α,2α,5,7-tetrahydroxy-1,2,3,4-tetrahydro-6,11-naphthacendion (Verbindung VIII) direkt durch Behandlung des 5,7-Dihydroxy-9-methoxy-2-methyl-3,4-dihydro-6,11-naphthacendions (Verbindung V) mit Osmiumtetroxyd $OsO_4$ erhalten.

Bei dieser Variante kann man ein einziges Isomer, nämlich 1α,2α, erhalten, aber dies verläuft mit kleinerer Ausbeute.

Nach einer weiteren vorteilhaften Ausführungsform des erfindungsgemässen Verfahrens erfolgt die Herstellung des Ketenacetals (1. Stufe) dadurch, dass man der Lösung das Lithiumamid, dann die Verbindung IV und anschliessend das Silylierungsmittel bei einer Temperatur zwischen -110°C und 0°C unter einer Inertgasatmosphäre zusetzt.

Nach einer weiteren vorteilhaften Ausführungsform des erfindungsgemässen Verfahrens verwendet man im Verlauf der 3. Stufe meta-Chlorperbenzoesäure als Persäure.

Die erfindungsgemäss im Verlauf der 4. Stufe eingesetzte Säure ist Trifluoressigsäure $CF_3COOH$.

Ebenfalls erfindungsgemäss erfolgt die Oxydation im Verlauf der 5. Stufe mittels eines Oxydationsmittels aus der $CrO_3$, $K_2Cr_2O_7$, Jones-Reagenz usw. umfassenden Gruppe.

Nach einer weiteren zweckmässigen Ausführungsform des erfindungsgemässen Verfahrens besteht das im Verlauf der 6. Stufe verwendete polare Lösungsmittel aus Pyridin und die starke, nicht-nukleophile Base aus Benzyltrimethylammoniumhydroxyd (Triton B).

Gegenstand vorliegender Erfindung sind ferner durch Glykosidierung von aus erfindungsgemässen Steffimycinonanalogen bestehenden Aglykonen erhaltene Antitumormittel.

Nach einer besonders vorteilhaften Ausführungsform des Gegenstands der Erfindung werden Aminozucker als Zucker für die Glykosidierung eingesetzt.

Ein weiterer Gegenstand der vorliegenden Erfindung ist ein Verfahren zur Glykosidierung von aus erfindungsgemässen Steffimycinonanalogen bestehenden Aglykonen, welches dadurch gekennzeichnet ist, dass man eine It-verbindung, deren freie OH-Funktionen mit para-Nitrobenzoylgruppen (PNB) und deren freie Aminfunktionen mit Trifluoracetylgruppen $-COCF_3$ substituiert sind, in einem geeigneten Lösungsmittel in Gegenwart einer katalytischen Menge para-Toluolsulfonsäure zur Reaktion bringt, das Reaktionsprodukt abtrennt, die PNB- und $-COCF_3$-Gruppen durch Hydrolyse abspaltet und das so erhaltene Reaktionsprodukt reinigt sowie gegebenenfalls in ein Salz einer pharmakologisch unbedenklichen Säure überführt.

Neben den obigen Ausführungsformen umfasst die Erfindung noch weitere, die aus der nachfolgenden Beschreibung zu entnehmen sind, die sich mit Herstellungsbeispielen für Zwischenprodukte der Steffimycinonanalogen und der Glykosidierung der erhaltenen erwünschten Aglykone sowie mit einem Bericht über die pharmakologische Untersuchung befasst.

Es versteht sich jedoch, dass diese Beispiele und dieser Bericht nur der Erläuterung des Erfindungsgegenstands dienen und diesen in keiner Weise einschränken.

HERSTELLUNGSBEISPIELE

1) Herstellung der Verbindung der Formel III ($R = C_2H_5$)
Zu einer Lösung von zuvor aus Diisopropylamin (2,5 ml, 0,0178 Mol) und nBuLi (10,75 ml 1,55m-Hexanlösung) hergestelltem Lithiumdiisopropylamid in wasserfreiem Tetrahydrofuran (THF) (8,3 ml) gibt man nacheinander bei -78°C unter Stickstoff in THF (1 ml) gelösten 2,4-Dimethyl-1,3-cyclohexadiencarbonsäureäthylester (IV) (3 g, 0,0166 Mol) und dann Chlortrimethylsilan (4,2 ml, 0,045 Mol).

Dann lässt man die Temperatur des Reaktionsgemischs unter Rühren allmählich auf 20°C ansteigen. Nach Entfernung der Lösungsmittel nimmt man den Rückstand in Hexan (ungefähr 20 ml) auf und zentrifugiert. Nach Abdampfen des Hexans erhält man dabei das Ketenacetal (III) (3,25 g, 77%) in Form eines blassgelben Oels.
. NMR ($CCl_4$): die chemischen Verschiebungen sind in ppm ($\delta$) mit Tetramethylsilan als Bezugsziffer angegeben.
0,17 ppm (Singlett, 9H); 1,23 ppm (Triplett, J = 7Hz, 3H); 1,7 ppm (Singlett, 3H); 3,77 ppm (Quadruplett, J = 7Hz, 2H); 4,73 ppm (Singlett mit Schulter, 1H); 5,03 ppm (Dublett, J = 2 Hz, 1H); 5,67 ppm (Singlett, 1H).

2) Herstellung des 5,7-Hydroxy-9-methoxy-2-methyl-3,4-dihydro-6,11-naphthacendions.

Zu einer bei 0°C gehaltenen Lösung von 3-Chlor-5-hydroxy-7-methoxy-1,4-naphthochinon (2,56 g, 0.0107 Mol) in wasserfreiem THF (120 ml) gibt man rasch das silylierte Ketenacetal (III) 3,25 g, 0,0129 Mol) in THF (40 ml). Nach 5 Stunden bei Raumtemperatur wird das Reaktionsgemisch in 0,1n-HCl-Lösung gegossen und dann bis zur Entfärbung der

wässrigen Phase mit Methylenchlorid extrahiert. Nach Trocknen über $Na_2SO_4$ und Eindampfen im Vakuum filtriert man den Rückstand über Kieselsäuregel (150 g) mit $CH_2Cl_2$ als Laufmittel. Dabei erhält man die Verbindung V (2 g, 60%) als orangefarbenes Pulver vom Schmelzpunkt 229-230°C.

. NMR ($CDCl_3$): 2,0 ppm (Singlett, 3H); 2,43 ppm (Triplett, J = 7Hz, 2H); 2,86 ppm (Triplett, J = 7Hz, 2H); 3,91 ppm (Singlett, 3H); 6,23 pp, (Singlett, 1H); 6,61 ppm (Singlett, 1H); 7,16 ppm (breites Singlett, 1H); 7,4 pp, (breites Singlett, 1H); 12,27 ppm (Singlett, 1H); 12,33 ppm (Singlett, 1H).

. IR (KBr-Scheibe): Die folgenden charakteristischen Banden sind in $cm^{-1}$ angegeben. 3275, 3030, 1680, 1640, 1620, 1600, 1480, 1450, 1420, 1390, 1300, 1215 und 1180.

. Massenspektrum: die relative Häufigkeit der Zacken ist jeweils in Klammern als Prozent angegeben.
m/z = 336 (57%); 334 (91%); 274 (68%); 238 (100%); 175 (57%); 133 (82%).

3) Herstellung des (±)-5,7-Dihydroxy-1,2-epoxy-9-methoxy-2-methyl-1,2,3,4-tetrahydro-6,11-naphthacendions (VII)

Das in der vorhergehenden Stufe erhaltene Produkt V (0,30 g, 0,89 mMol) wird im kleinstmöglichen Volumen Methylenchlorid (ungefähr 25 ml) aufgelöst. Man kühlt auf 0°C ab und versetzt dann langsam mit meta-Chlorperbenzoesäure (0,46 g, 2,70 mMol). Nach 12 Stunden bei Raumtemperatur wird das Reaktionsgemisch in eine gesättigte Natriumsulfitlösung gegossen und dann mit Methylenchlorid extrahiert. Nach Trocknen und Eindampfen des Lösungsmittels filtriert man den Rückstand über Kieselsäuregel (30 g) mit $CH_2Cl_2$ als Laufmittel. Dabei gewinnt man das Epoxyd VII (0,22 g, 70%) als gelbes Pulver vom Schmelzpunkt 233-234°C.

. NMR ($CDCl_3$): 1,55 (Singlett, 3H); 3,66 (Singlett, 1H); 3,9 ppm (Singlett, 3H); 6,64 ppm (Singlett bei Entkopplung, 1H); 7,33 ppm (Dublett, J = 2 Hz, 1H); 7,79 ppm (Singlett, 1H); 12,2 ppm (Singlett, 1H); 12,43 ppm (Singlett, 1H).

. IR (KBr-Scheibe): 3300, 3280, 2920, 2960, 1680, 1620, 1605, 1480, 1445, 1405, 1395, 1290, 1270, 1235, 1210, 1165

und 1110.

. Massenspektrum: 352 (47%); 337 (23%); 334 (26%); 324 (100%); 309 (23%); 284 (10%); 69 (38%); 55 (61%).

4) Herstellung der Zwischenprodukte VIII und IX

Unter Stickstoff und bei Raumtemperatur versetzt man eine Lösung des Epoxyds VII (0,1 g; 0,28 mMol) in Methylenchlorid (60 ml) mit Trifluoressigsäure (0,097 g; 0,85 mMol). Das Gemisch wird dann noch 12 Stunden gerührt. Nach Abdampfen des Lösungsmittels nimmt man das rohe Reaktionsgemisch in THF (60 ml) auf und hydrolysiert es mit gesättigter Natriumbicarbonatlösung, bis das Ausgangsprodukt völlig verschwunden ist (durch Dünnschichtchromatographie (DSC) auf Merck-Kieselgelplatte verfolgt).

Man extrahiert das Gemisch der Isomeren 1α,2α und 1β,2α (0,068 g, 0,18 mMol) mit Methylenchlorid und trocknet über Natriumsulfat. Nach Abdampfen des Lösungsmittels löst man den Rückstand in Aceton (70 ml) wieder auf und versetzt unter Stickstoff und bei Raumtemperatur mit Jones-Reagenz (0,3 ml, 0,57 mMol). Man rührt für 10 Stunden, hydrolysiert dann und extrahiert mit $CH_2Cl_2$. Nach Trocknen über Natriumsulfat wird die Verbindung IX über Kieselgel (30 g) unter Verwendung eines Gemischs aus Methylenchlorid und Methanol (1%) als Laufmittel filtriert. Dabei erhält man das Produkt IX (0,052 g, 0,14 mMol) als gelbes Pulver vom Schmelzpunkt 245-250°C.

. Ir (KBr-Scheibe): 3360, 3000, 2960, 1700, 1680, 1640, 1620, 1450, 1430, 1400, 1320, 1250, 1160, 1110, 1035, 970 und 790.

. Massenspektrum: m/z = 368 (58%); 340 (52%); 325 (100%); 310 (17%); 297 (52%); 282 (20%).

. UV (Aethanol): die $\lambda_{max}$ sind in Nanometern angegeben.

$\lambda_{max}$ = 240 nm (log ε = 4,46)
= 285 nm (log ε = 4,43)
= 440 nm (log ε = 4,23)

5) Herstellung des 8-Desmethoxy-analogen (II)

Man löst das Hydroxyketon IX (0,077 g, 0,21 mMol) in Pyridin (10 ml) und versetzt bei 0°C mit 10%iger Triton-B-Lösung in Pyridin. Die Umsetzung wird durch DSC verfolgt, bis das Ausgangsprodukt völlig verschwunden ist (ungefähr 4 Stunden). Nach Hydrolyse mit 1n-HCl-Lösung extrahiert man das rohe Reaktionsgemisch mit $CH_2Cl_2$ und trocknet über $Na_2SO_4$.

Nach Chromatographie auf einer präparativen Platte gewinnt man die Verbindung II als gelbes Pulver (0,048 g, 0,12 Mol) vom Schmelzpunkt 223-225°C.

. IR (KBr-Scheibe): 3360, 3020, 2970, 1710, 1680, 1620, 1420, 1400, 1320, 1290, 1260, 1205, 1170, 1115, 1040, 970 und 740.

. Massenspektrum: m/z = 384 (17%); 368 (48%); 366 (42%); 350 (90%); 338 (64%); 324 (100%); 297 (44%); 277 (37%); 270 (33%).

. UV (Aethanol): die $\lambda_{max}$ sind in Nanometern angegeben.

$\lambda_{max}$ = 240 nm (log ε = 4,38)
= 233 nm (log ε = 4,21)
= 455 nm (log ε = 3,95)

6) Glykosidierungsbeispiel

Man löst die racemische Verbindung II in der kleinstmöglichen Menge Benzol bei Raumtemperatur in Gegenwart einer katalytischen Menge para-Toluolsulfonsäure und versetzt dann mit 3 Aequivalenten der Verbindung der nachfolgenden Formel X:

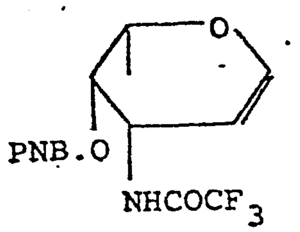

X

(1,5-Anhydro-2,3,6-tridesoxy-$O^4$-paranitrobenzoyl-3-trifluoracetamido-L-lyxo-1-hexenit). Man lässt das Reaktionsgemisch unter Rühren sowie unter Stickstoff 48 Stunden bei

Raumtemperatur stehen.

Nach Abdampfen des Lösungsmittels chromatographiert man den Rückstand auf einer Kieselgelplatte unter Verwendung eines Gemischs aus Benzol und Essigester als Laufmittel. Dabei wird die nachfolgende Verbindung XI abgetrennt:

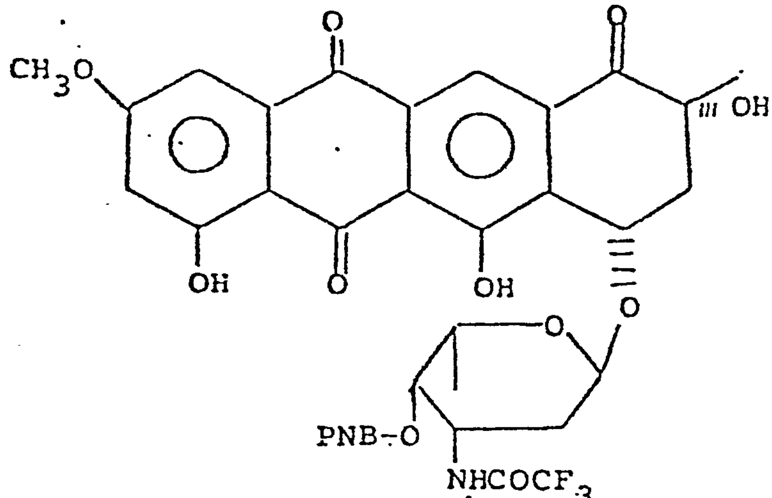

XI

((2R,4S)-9-Methoxy-2-methyl-$O^4$-(2',3',6'-tridesoxy-$O^{4'}$-paranitrobenzoyl-3'-trifluoroacetamido-α-L-lyxo-hexapyranosyl)-2,4,5,7-tetrahydroxy-3,4-dihydro-2H-1,6,11-naphthacentrion) (M = 758, $C_{35}H_{29}F_3N_2O_{14}$) in Form eines gelben Pulvers.

Man löst das Glykosid XI in überschüssigem Dioxan und versetzt mit wässriger 0,1m-Natronlauge. Nach 2 Stunden bei $0^o$ wird der pH mit 0,1m-HCl-Lösung wieder auf 6 gestellt. Nach Extraktion mit Methylenchlorid stellt man die wässrige Phase wieder auf pH 8 und extrahiert nochmals mit Methylenchlorid.

Dabei erhält man die nachfolgende Verbindung XII:

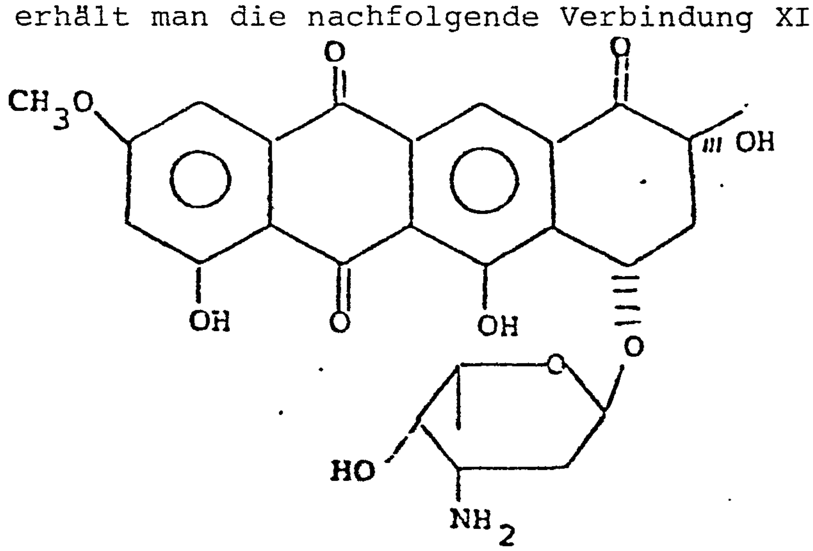

XII

 

als gelbes Pulver.

(2R,4S)-9-Methoxy-2-methyl-$O^4$-(3'-amino-2',3',6'-tridesoxy-α-L-lyxo-hexapyranosyl)-2,4,5,7-tetrahydroxy-3,4-dihydro-2H-1,6,11-naphthacentrion, $C_{26}H_{27}NO_{10}$, M = 513.

Man löst die Verbindung XII in Chloroform und behandelt die so erhaltene Lösung dann mit einem Moläquivalent äthanolischer Salzsäure. Durch Aetherzugabe wird das entsprechende Hydrochlorid ausgefällt. $C_{26}H_{28}ClNO_{10}$, M = 549,5.

PHARMAKOLOGISCHER BERICHT : BAKTERIZIDE WIRKUNG

Das Produkt der Formel II wurde an folgenden Mikroorganismen geprüft:

Bacillus pumilus (CNCM 7618) und
Bacillus subtilis (ATCC 6533)

Es wurde auf eine Konzentration von 100 µg/ml in einem Gemisch aus 60% Dimethylsulfoxyd und 40% destilliertem Wasser solubilisiert. Nach 24 Stunden wurde festgestellt, dass das Wachstum der beiden Mikroorganismen völlig gehemmt war.

Wie aus dem obigen ersichtlich ist, beschränkt sich die Erfindung keineswegs auf solche Ausführungsformen, Realisierungen und Anwendungen wie oben näher beschrieben, sondern umfasst sämtliche Varianten, die einem Fachmann naheliegen könnten, ohne vom Umfang oder dem Gedanken der Erfindung abzuweichen.

Patentansprüche:

1. Steffimycinonanaloge, dadurch gekennzeichnet, dass sie aus 8-entmethoxylierten Derivaten der nachfolgenden Formel II bestehen:

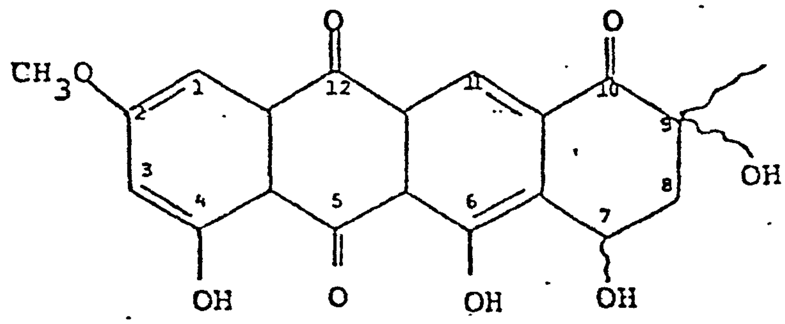

II

2. Steffimycinonanalog nach Anspruch 1, dadurch gekennzeichnet, dass es aus (±)-8-Desmethoxy-steffimycinon besteht.

3. Steffimycinonanalog nach Anspruch 1, dadurch gekennzeichnet, dass es aus (±)-7-Epi-8-desmethoxy-steffimycinon besteht.

4. Verfahren zur Herstellung von Steffimycinonanalogen der allgemeinen Formel II nach Anspruch 1, gekennzeichnet durch die Kombination der folgenden Stufen:
1) Herstellung des silylierten Ketenacetals der nachfolgenden allgemeinen Formel III:

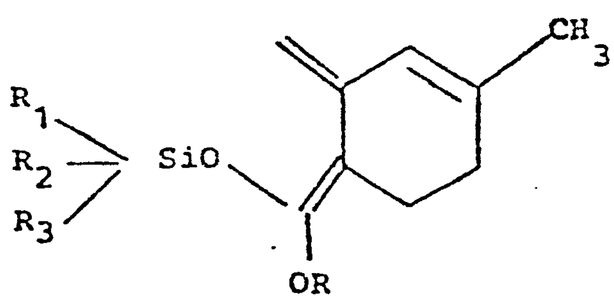

III

worin

R eine gegebenenfalls substituierte Alkyl- oder Arylgruppe sowie

$R_1$, $R_2$ und $R_3$, die gleich oder verschieden sein können, eine Alkylgruppe darstellen,

durch Umsetzung eines Lithiumamids und eines Silylierungsmittels mit einem Alkyl- oder Arylester der 2,4-Dimethyl-1,3-cyclohexadiencarbonsäure der nachfolgenden

allgemeinen Formel IV:

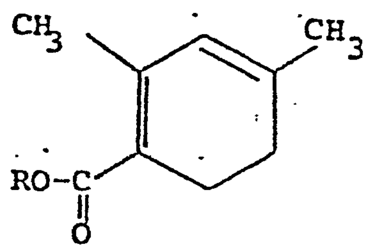

IV

wobei R die oben angegebene Bedeutung hat,

2) Herstellung des 5,7-Dihydroxy-9-methoxy-2-methyl-3,4-dihydro-6,11-naphthacendions der nachfolgenden Formel V:

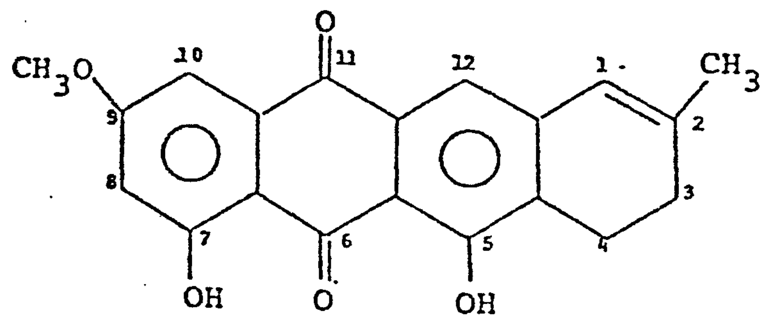

V

durch Umsetzung der Verbindung III mit einem 5-Hydroxy-7-methoxy-1,4-naphthochinon der nachfolgenden Formel VI:

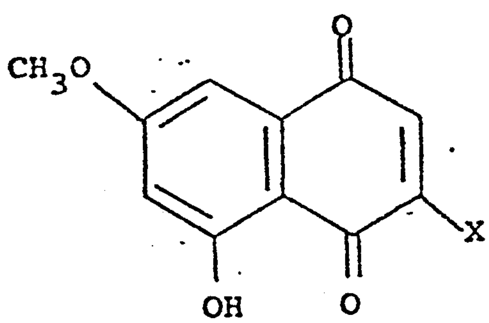

VI

worin

X für ein Halogenatom oder Wasserstoffatom steht,

3) Herstellung des (±)-5,7-Dihydroxy-1,2-epoxy-9-methoxy-2-methyl-1,2,3,4-tetrahydro-6,11-naphthacendions der nachfolgenden Formel VII:

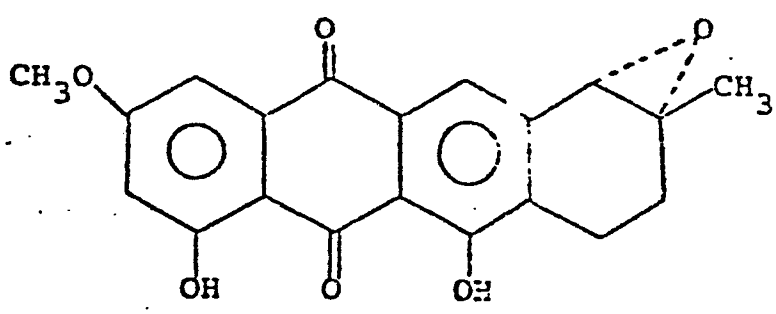

VII

durch Einwirkung einer Persäure auf das Olefin der

Formel V,

4) Herstellung eines Gemischs der racemischen Diole [1α,2α und 1β,2α] der nachfolgenden Formel VIII:

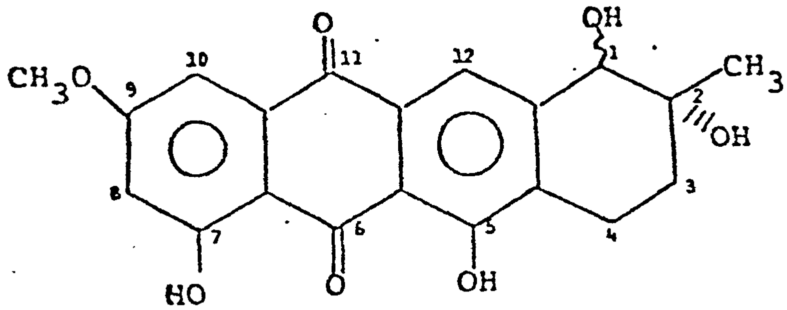

VIII

durch Behandlung des in der vorhergehenden Stufe erhaltenen Epoxyds mit einer Säure,

5) Herstellung des (±)-9-Methoxy-2-methyl-2,5,7-trihydroxy-3,4-dihydro-2H-1,6,11-naphthacentrions der nachfolgenden Formel IX:

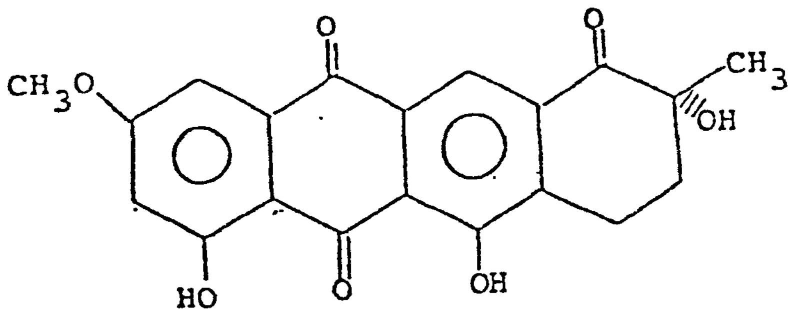

IX

durch Oxydation des in der vorhergehenden Stufe erhaltenen Gemischs aus

(±)-9-Methoxy-2-methyl-1α,2α,5,7-tetrahydroxy-1,2,3,4-tetrahydro-6,11-naphthacendions und

(±)-9-Methoxy-2-methyl-1β,2α,5,7-tetrahydroxy-1,2,3,4-tetrahydro-6,11-naphthacendions, und

6) Herstellung von 8-Desmethoxysteffimycinonanalogen der allgemeinen Formel II durch Behandlung des Produkts IX in einem polaren Lösungsmittel mit einer starken nichtnukleophilen Base und nachfolgende saure Hydrolyse.

5. Verfahren nach Anspruch 4, dadurch gekennzeichnet, dass das (+)-9-Methoxy-2-methyl-1α,2α,5,7-tetrahydroxy-1,2,3,4-tetrahydro-6,11-naphthacendion (Verbindung VIII) direkt durch Behandlung des 5,7-Dihydroxy-9-methoxy-2-methyl-3,4-dihydro-6,11-naphthacendions (Verbindung V) mit Osmiumtetroxyd $OsO_4$ erhalten werden kann.

6. Verfahren nach Anspruch 4, dadurch gekennzeichnet, dass die Herstellung des Ketenacetals (1. Stufe) dadurch erfolgt, dass man der Lösung von Lithiumamid nacheinander die Verbindung IV und dann das Silylierungsmittel bei einer Temperatur zwischen $-110^{o}C$ und $0^{o}C$ und unter einer Inertgasatmosphäre zusetzt.

7. Verfahren nach Anspruch 4, dadurch gekennzeichnet, dass man als Persäure im Verlauf der 3. Stufe meta-Chlorperbenzoesäure einsetzt.

8. Verfahren nach Anspruch 4, dadurch gekennzeichnet, dass man als Säure im Verlauf der 4. Stufe Trifluoressigsäure $CF_3COOH$ einsetzt.

9. Verfahren nach Anspruch 4, dadurch gekennzeichnet, dass man die Oxydation im Verlauf der 5. Stufe mittels eines Oxydationsmittels aus der $CrO_3$, $K_2Cr_2O_7$ und Jones-Reagenz umfassenden Gruppe durchführt.

10. Verfahren nach Anspruch 4, dadurch gekennzeichnet, dass das im Verlauf der 6. Stufe eingesetzte polare Lösungsmittel aus Pyridin und die starke nicht-nukleophile Base aus Benzyltrimethylammoniumhydroxyd (Triton B) besteht.

11. Anwendung der Steffimycinonanalogen der allgemeinen Formel II nach einem der Ansprüche 1 bis 3 als Bakterizide.

12. Verwendung der Steffimycinonanalogen der allgemeinen Formel II nach einem der Ansprüche 1 bis 3 als Aglykone zur Herstellung von Antitumormitteln.

13. Antitumormittel, gebildet durch Glykosidierung der Steffimycinonanalogen der Formel II nach einem der Ansprüche 1 bis 3.

14. Antitumormittel nach Anspruch 13, gebildet durch Glykosidierung mittels Aminozuckern.

15. Antitumormittel nach Anspruch 14, dadurch gekennzeichnet, dass es aus (2R,4S)-9-Methoxy-2-methyl-$O^4$-(3'-amino-2',3',6'-tridesoxy-α-L-lyxo-hexapyranosyl)-2,4,5,7-tetrahydroxy-3,4-dihydro-2H-1,6,11-naphthacentrion der nachfolgenden Formel XII:

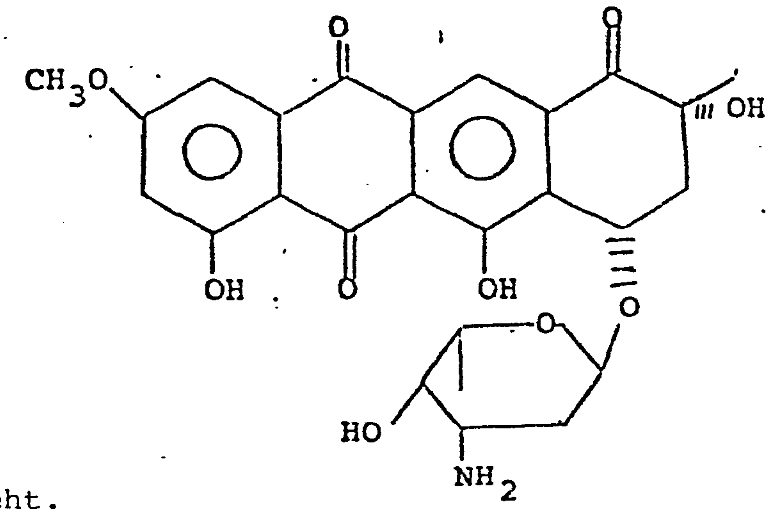

XII

besteht.

16. Verfahren zur Glykosidierung von aus Steffimycinonanalogen nach einem der Ansprüche 1 bis 3 bestehenden Aglykonen, dadurch gekennzeichnet, dass man eine It-verbindung, deren freie OH-Funktionen mit para-Nitrobenzoylgruppen (PNB) und deren freie Aminofunktionen mit Trifluoracetylgruppen $-COCF_3$ substituiert sind, in einem geeigneten Lösungsmittel in Gegenwart einer katalytischen Menge para-Toluolsulfonsäure zur Reaktion bringt, das Reaktionsprodukt abtrennt, die PNB- und $-COCF_3$-Gruppen durch Hydrolyse abspaltet und das so erhaltene Reaktionsprodukt reinigt sowie gegebenenfalls in ein Salz einer pharmakologisch unbedenklichen Säure überführt.

17. Verbindung der nachfolgenden Formel III:

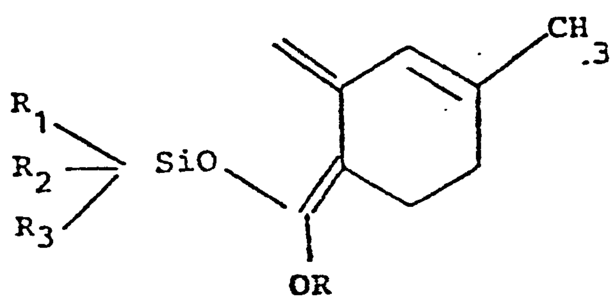

III

worin

R eine gegebenenfalls subsituierte Alkyl- oder

Arylgruppe sowie

$R_1$, $R_2$ und $R_3$, die gleich oder verschieden sein können, eine Alkylgruppe darstellen,

als neues Zwischenprodukt zur Synthese von Steffimycinonanalogen nach einem der Ansprüche 1 bis 3.

18. Racemische Verbindung der nachfolgenden Formel V:

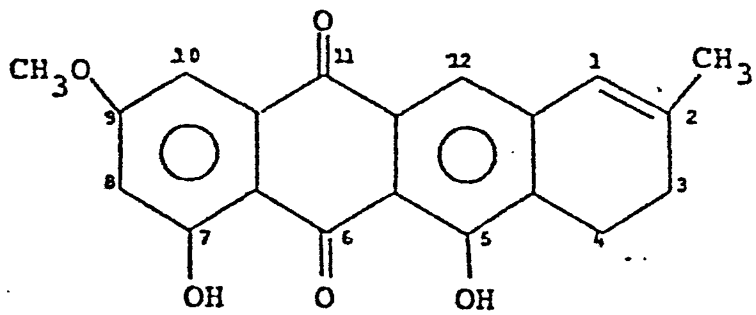

V

als neues Zwischenprodukt zur Synthese von Steffimycinonanalogen nach einem der Ansprüche 1 bis 3.

19. Racemische Verbindung der nachfolgenden Formel VII:

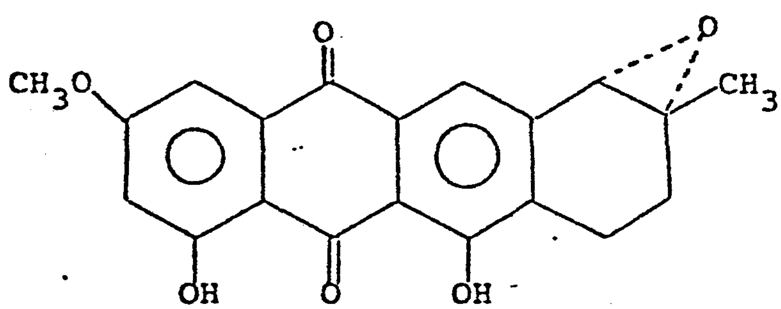

VII

als neues Zwischenprodukt zur Synthese von Steffimycinonanalogen nach einem der Ansprüche 1 bis 3.

20. Racemische Verbindung der nachfolgenden Formel VIII:

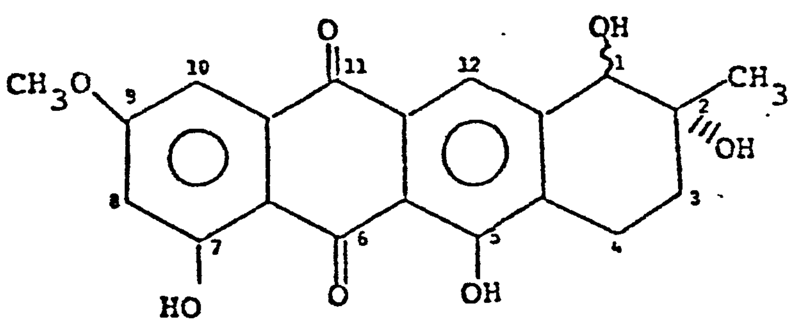

VIII

als neues Zwischenprodukt zur Synthese von Steffimycinonanalogen nach einem der Ansprüche 1 bis 3.

21. Racemische Verbindung der nachfolgenden Formel IX:

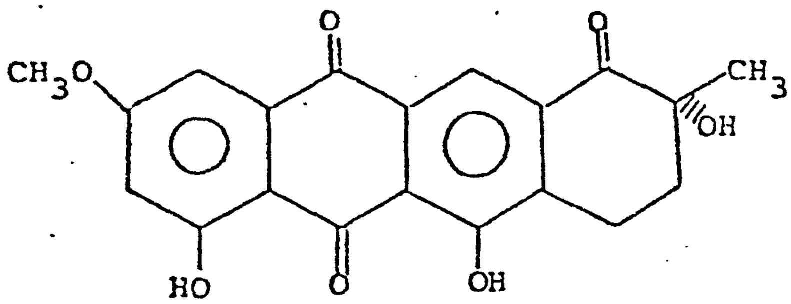

IX

als neues Zwischenprodukt zur Synthese von Steffimycinonanalogen nach einem der Ansprüche 1 bis 3.